## Europäisches Patentamt

## European Patent Office

## Office européen des brevets

(19)

(11) Veröffentlichungsnummer: **0 168 691**
A1

# (12) EUROPÄISCHE PATENTANMELDUNG

(21) Anmeldenummer: 85107986.3

(22) Anmeldetag: **27.06.85**

(51) Int. Cl.⁴: **A 61 B 5/10,** A 61 B 5/05,
G 01 N 24/04

(30) Priorität: 05.07.84 DE 3424788

(43) Veröffentlichungstag der Anmeldung: **22.01.86**
**Patentblatt 86/4**

(84) Benannte Vertragsstaaten: **DE GB NL**

(71) Anmelder: **Siemens Aktiengesellschaft, Berlin und München Wittelsbacherplatz 2, D-8000 München 2 (DE)**

(72) Erfinder: **Wirth, Axel, Amselstrasse 31, D-8551 Hemhofen (DE)**
Erfinder: **Schneider, Siegfried, Dr., Kulmbacher Strasse 33, D-8520 Erlangen (DE)**

(54) **Vorrichtung zur berührungslosen Messung von Organbewegungen.**

(57) Ein Meßsender (11, 17) erzeugt ein HF-Dauersignal, welches auf eine Meßspule (2) gegeben wird. Die Meßspule (2) befindet sich in einem Raum (3), der auch zur Aufnahme eines Patienten (7) dient. Die Meßspule (2) ist Bestandteil eines Schwingkreises (1), welcher an eine Meßvorrichtung (9) zur Messung von Impedanzänderungen angeschlossen ist. Aufgrund der internen Masseverschiebungen im Patienten (7), z.B. der Lungenbewegung, ändern sich die Verhältnisse im Raum (3); damit ändert sich auch die Dämpfung des Schwingkreises (1). Das Ausgangssignal der Meßeinrichtung (9) ist ein Maß für die Organbewegungen des ruhenden Patienten (7). Besonders vorteilhaft ist die Vorrichtung, wenn sie zusammen mit einem NMR-Gerät (13) angewendet wird. Die NMR-Bildaufnahme läßt sich hier mit den Organbewegungen des Patienten steuern. Der Meßsender (11) und die Meßspule (2) können zugleich die entsprechenden HF-Teile (17, 23), die ohnehin im NMR-Gerät (13) enthalten sind, sein.

0168691

Siemens Aktiengesellschaft
Berlin und München

Unser Zeichen
VPA 84 P 3 2 4 7 E

Vorrichtung zur berührungslosen Messung von Organbewegungen

Die Erfindung betrifft eine Vorrichtung zur berührungslosen Messung der Bewegungen an einem ruhenden Patienten, insbesondere zur Messung von Organbewegungen, die
durch Atmung oder Herzschlag verursacht sind.

Vorrichtungen dieser Art werden z.B. zur Patientenüberwachung verwendet. Mit ihnen läßt sich der Atemrhythmus
eines Patienten oder die Herztätigkeit desselben feststellen. Unregelmäßigkeiten oder kritische Ereignisse
können rechtzeitig erkannt werden, um Gegenmaßnahmen
einzuleiten. Außerdem werden Vorrichtungen der genannten Art bei Bildaufnahmesystemen eingesetzt, um den Auslösezeitpunkt für die Bildaufnahme mit den Organbewegungen des Patienten abzustimmen.

Eine Vorrichtung der eingangs genannten Art ist in dem
Artikel "Monitoring Variations of Biological Impedances
at Microwave Frequencies", IEEE Transactions on Biomedical Engineering, Vol. BME-30, No. 9, Sept. 1983, beschrieben. Es wird dort eine Vorrichtung vorgestellt,
bei der eine trichterförmige Antenne direkt auf die
Oberfläche des Patienten aufgesetzt wird, um die Mikrowellenimpedanz des darunterliegenden Gewebes zu bestimmen. Die Mikrowellenimpedanz ändert sich mit den Bewegungen, die im Inneren des Patienten vorliegen. Es wird
auf diese Weise die Bewegung von Blutgefäßen dicht unterhalb der Haut untersucht. Nachteilig bei dieser Vorrichtung ist, daß ein wesentlicher mechanischer Teil,

Wil 2 Ler / 02.07.1984

nämlich die Antenne, direkt am Patienten appliziert werden muß. Weiterhin ist nachteilig, daß der Untersuchungsraum relativ beschränkt ist. Bewegungen größeren Ausmaßes lassen sich mit der dort vorgestellten Vorrichtung nur schwer erfassen.

Eine Vorrichtung der genannten Art wurde auch bereits bei klinischen Untersuchungen zur Triggerung von Bildaufnahmen eingesetzt, wie in "Technicare News", RSNA Special Edition, Nov. 1983, beschrieben ist. Dort sind NMR-Aufnahmen dargestellt, die sowohl unter Berücksichtigung der Herz- als auch der Lungenbewegungen zustandegekommen sind. Es wird offengelassen, mit welchen Mitteln die Lungenbewegung oder die Herzbewegungen registriert wurden.

Zur Eliminierung der Zwerchfell- und Lungenbewegung bei der NMR-Bildaufnahme wurde bereits von F.S. Prato et al. auf dem 2nd Annual Meeting of the Society of Magnetic Resonance in Medicine, August 16-19, 1983, San Francisco, Seiten 284 bis 285, vorgestellt, den Patienten erhöhte Sauerstoffmengen und Narkosegas zuzuführen, um anschliessend die Atemtätigkeit für fünf Minuten anzuhalten. Alternativ hierzu wurde ebenfalls vorgetragen, den Luftstrom mittels eines Pneumotachographen zu messen oder zu registrieren.

Besondere Bedeutung gewinnt eine Vorrichtung zur Bewegungsmessung beim Einsatz zur Triggerung einer Bildaufnahme. In der NMR-Technik werden z.B. für eine einzige Bildaufnahme mehrere Minuten benötigt. Die Aufnahmezeiten können bis zu einer dreiviertel Stunde dauern. Die gewonnenen Bilddaten werden in einem komplizierten Rechenverfahren zu einem Schnittbild zusammengesetzt. Fehlinformationen, wie sie aufgrund einer Bewegung des Patienten zustandekommen, führen zu starken Verzerrungen und können ein NMR-Bild unbrauchbar machen. Ebenso führen die Bewegungen des Herzens, der Lunge und anderer Organe oder benachbarter Körperteile zu einer Spiegelung, Schattenbildung und Unschärfe, die die Bildqualität negativ beeinflußt. Man ist daher dazu übergegangen, nur solche Bilddaten zu verwenden oder aufzunehmen, die immer zum gleichen (äquivalenten) Zeitpunkt einer Bewegungsphase erfolgen. So z.B. wird bei Aufnahmen in der Herzgegend in der Regel die R-Zacke des Elektrokardiogramms zum Auffinden des Auslösezeitpunktes für die Bildaufnahme herangezogen. Für Aufnahmen in der Lungengegend ist man auch schon dazu übergegangen, die Bildaufnahme mit der Lungenbewegung zu synchronisieren.

Bei den herkömmlichen Vorrichtungen zur Erfassung der Bewegungstätigkeit eines Patienten sind in der Regel Handhabungen am Patienten nötig. So z.B. wird ihm zur Bewegungserfassung der Lunge an der Nase ein Temperatur- oder Druckfühler angebracht, der die Temperatur oder den Druck der ausgeatmeten Luft mißt. Aus den registrierten Werten werden Rückschlüsse auf die Bewegungen der Lunge gezogen. Eine andere bekannte Vorrichtung macht sich die Bewegung der Bauchdecke beim Atmen zunutze. Mittels eines Klebestreifens wird ein Luftkissen an der Bauchdecke befestigt. Aufgrund der Atembewegung wird das im Luftkissen eingeschlossene Luftvolumen mehr oder weniger komprimiert. Das resultierende Drucksignal dient zur Ableitung einer Information über die Atembewegung. In einer weiteren bekannten Vorrichtung werden zur Ermittlung der Herzbewegung EKG-Elektroden auf der Brust des Patienten angebracht.

Nachteilig bei diesen Vorrichtungen ist, daß Teile oder Zuleitungen am Patienten befestigt werden müssen. Außerdem sind Zuleitungen vorhanden, die den Patienten behindern und auch die Bildaufnahme beeinträchtigen können. Weiterhin ist nachteilig, daß nicht mit einer einzigen Vorrichtung eine Vielzahl an Organbewegungen erfaßt werden kann, sondern für jede Organbewegung ein separates Gerät zu installieren ist.

Aufgabe der Erfindung ist es daher, eine Vorrichtung der eingangs genannten Art anzugeben, die einen bequemen, von Zuleitungen zu dem Patienten freien Einsatz ermöglicht.

Diese Aufgabe wird erfindungsgemäß dadurch gelöst, daß eine mit einem HF-Meßsender verbundene HF-Meßspule Bestandteil eines HF-Schwingkreises ist, daß an der HF-Meßspule ein zur Aufnahme des Patienten bestimmter Raum vorgesehen ist, und daß eine Meßeinrichtung an dem Schwingkreis angeschlossen ist, an deren Signalausgang ein Meßsignal abgreifbar ist, welches dann, wenn der Patient in den besagten Raum eingebracht ist, eine Änderung der Dämpfung des Schwingkreises aufgrund einer Masseverschiebung des Patienten anzeigt. Um die Leistung des HF-Senders optimal auszunutzen, sollte die Impedanz der HF-Meßspule an den HF-Meßsender leistungsmäßig angepaßt sein.

Vorteil der Vorrichtung ist, daß Anschlüsse am Patienten und Zuleitungen zu solchen Anschlüssen nicht benötigt werden. Darüber hinaus können mehrere Organbewegungen, so z.B. die der Lunge, des Herzens, des Zwerchfells oder der Blutgefäße, gleichzeitig erfaßt werden. Aufgrund der unterschiedlichen Frequenzen und Amplituden, mit denen sich die verschiedenen Organe bewegen, läßt sich das Meßsignal so weiterverarbeiten, daß mehrere Organbewegungen gleichzeitig ausgewertet werden können. Wie erwähnt, kann dies alles vorgenommen werden, ohne daß an dem Patienten eine Apparatur befestigt werden muß. Es entstehen so keine Probleme mit der Applikation von Aufnehmern am Patienten, mit deren Reinigen, Desinfizieren und mit deren Sterilisieren.

Besonders vorteilhafte Eigenschaften ergeben sich, wenn die Vorrichtung einem NMR-Gerät mit einer HF-Sende- und -Empfangsspule und mit einem HF-Sender zugeordnet ist. Hier ist es von besonderem Vorteil, wenn der HF-Meßsender und die HF-Meßspule durch den HF-Sender bzw. die HF-Spule des NMR-Gerätes gebildet werden.

Der besondere Vorteil beim Einsatz in Verbindung mit einem NMR-Gerät besteht darin, daß ein Großteil der Bauteile bereits vorhanden ist, was eine erhebliche Kostenersparnis zur Folge hat. Bei der Kernspintomographie läßt sich, wie bereits erwähnt, mit dem Atemsignal des Patienten die NMR-Bilddatenerfassung oder -verarbeitung steuern. Weitere Vorteile ergeben sich, wenn nicht eine einzige HF-Spule für die Erfassung der Impedanz oder Impedanzänderung des Patienten vorgesehen ist, sondern wenn dazu eine Mehrzahl von HF-Spulen räumlich parallel nebeneinander angeordnet ist. Dann nämlich kann man diejenige Spule auswählen und z. B. zur Triggerung der Bildaufnahme heranziehen, welche die interessierende Organbewegung am deutlichsten darstellt. Auch ist es denkbar, zwei Spulen gleichzeitig auszunutzen, wobei die eine dann z.B. die Bewegungen, die durch das Herz verursacht werden, erfaßt, um die Synchronisation mit der Herzbewegung zu erzielen, und wobei die andere Spule z.B. die Bewegungen in der Zwerchfellgegend erfaßt, die dann für die Synchronisation mit der Zwerchfellbewegung herangezogen wird.

Weitere Ausgestaltungen und Vorteile der Vorrichtung ergeben sich aus der folgenden Beschreibung von sechs Figuren in Verbindung mit den Unteransprüchen. Es zeigen:

Fig. 1 eine Anordnung von Schwingkreis und zugeordnetem Raum zur Aufnahme eines Patienten in prinzipieller Darstellung,

Fig. 2 ein Ersatzschaltbild der Anordnung gemäß Figur 1,

Fig. 3 eine schematische Darstellung einer Vorrichtung nach Figur 1, die einem NMR-Gerät zugeordnet ist,

Fig. 4 eine schematische Darstellung einer Vorrichtung nach Figur 1 für ein NMR-Gerät, bei der HF-Meßspule und NMR-Sendespule identisch sind,

Fig. 5 ein Diagramm zur Erläuterung der Bildauslösung mit den Vorrichtungen nach den Figuren 3 und 4,

Fig. 6 eine Vorrichtung nach Figur 1 für ein NMR-Gerät, wobei HF-Sender und Meßsender sowie HF-Spule und Meßspule jeweils gemeinsam verwendet werden, und

Fig. 7 ein Diagramm zur Erläuterung der Bildauslösung mit der Vorrichtung nach Figur 6.

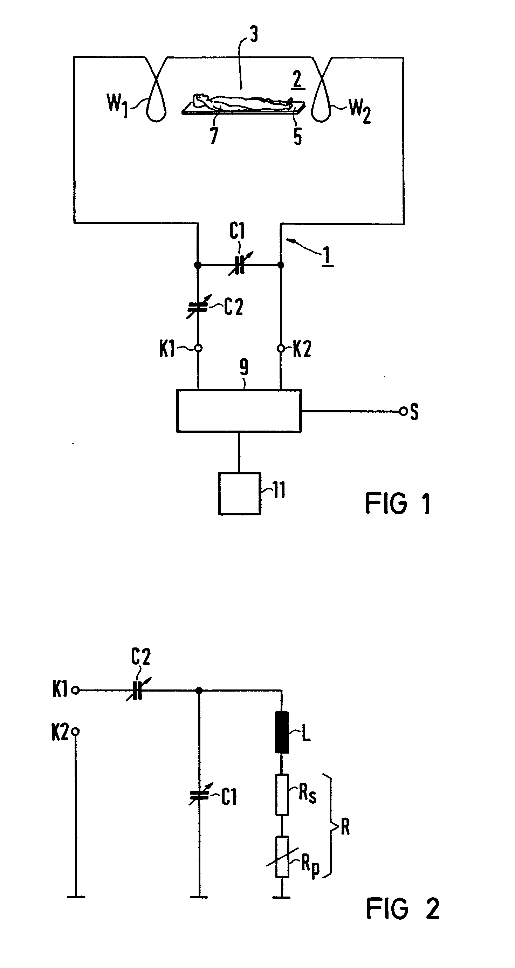

In den Figuren 1 und 2 ist ein Schwingkreis 1 dargestellt, der aus einer Spule 2 der Induktivität L mit zwei Teilwicklungen $W_1$ und $W_2$, einem Widerstand R und

zwei Kondensatoren C1, C2 mit veränderbaren Kapazitäten besteht. Der Widerstand R besteht aus den Teilwiderständen $R_S$ (ohm'scher Widerstand der Spule, konstant) und $R_P$ (Dämpfung durch den Patienten). Die Spule 2 und der Widerstand R liegen im Ersatzschaltbild in Reihe zueinander. Parallel zu dieser Reihenschaltung liegt der Kondensator C1 mit verstellbarer Kapazität. In Reihe zu dieser Parallelschaltung wiederum liegt der Kondensator C2 mit ebenfalls einstellbarer Kapazität. An den Ausgangsklemmen K1 und K2 dieser Anordnung ist eine Meßeinrichtung 9 angeschlossen. Die Spule 2 ist so dimensioniert, daß von ihr ein Raum 3 gebildet wird, in welchem ein Patientenlagerungstisch 5 Platz findet. Auf dem Patientenlagerungstisch 5 befindet sich ein ruhender Patient 7.

Von den Klemmen K1, K2 führen Leitungen in die Meßeinrichtung 9. Die Meßeinrichtung 9 ist geeignet, die Impedanz und die Änderungen der Impedanz des Schwingkreises 1 festzustellen. Ein Meßsender 11 speist über die Meßeinrichtung 9 ein Hochfrequenzsignal in die Meßspule 2 ein. Dieses Hochfrequenzsignal ist ein Dauersignal, vorteilhafterweise im Bereich von 20 MHz, z.B. von 21 MHz. Würde der Schwingkreis 1 durch keinerlei äußeren oder inneren Einfluß gestört, so bliebe die Dämpfung und damit die Impedanz des Schwingkreises 1 konstant, und von der Meßeinrichtung 9 würde ein konstantes Meßsignal S abgegeben.

Nun ist aber wegen der inneren Organbewegungen des Patienten 7 ein Einfluß auf den Schwingkreis 1 gegeben, der die Dämpfung der Spule 2 verändert. Aufgrund der Masseverschiebungen, die mit den Organbewegungen Hand in Hand gehen, ändert sich das Umgebungsfeld der Spule 2, d.h. im dargestelltem Beispiel die Impedanzverteilung im Spulenraum 3, wodurch die Dämpfung der Spule 2 verändert wird. Diese Veränderung wird von der Meßeinrichtung 9 erfaßt. Sie steht als Meßsignal S, welches ein Maß für die Organbewegungen im Patienten 7 und/oder für dessen Körperbewegung ist, zur Verfügung.

Je nach Empfindlichkeit des Schwingkreises 1 lassen sich schon geringe Masseverschiebungen wahrnehmen. Untersuchungen haben ergeben, daß dann, wenn der Schwingkreis 1 z.B. auf eine Impedanz von 50 Ohm eingestellt wurde, sich Schwankungen aufgrund der Organbewegungen im Patienten von bis zu 3 Ohm ergeben. Bei dem Meßsignal S handelt es sich um ein Summensignal, verursacht durch die Bewegungen verschiedener Organe. Enthalten diese Bewegungen unterschiedliche Frequenzanteile, so können die einzelnen Organbewegungen aus dem Meßsignal S bestimmt werden. Die Atembewegung ist z.B. relativ niederfrequent und in ihrer Amplitude relativ hoch, und zwar im Vergleich zur Herzbewegung, die in ihrer Frequenz deutlich höher, in ihrer Amplitude aber geringer liegt. Die Ableitung kann somit mittels frequenzsensitive Baugruppen erfolgen.

Die Vorrichtung bietet also den Vorteil, aus dem einzigen anfallenden Meßsignal S eine Vielzahl von Organbewegungen ableiten zu können. Dies ist verbunden mit einem weiteren wesentlichen Vorteil, der darin besteht, daß am Patienten 7 selber keinerlei Handhabungen vorzunehmen sind. Es werden keine zusätzlichen Teile, Elektroden, Anschlüsse, Kabel od.dgl. benötigt, die - wie Leibbinden - jedesmal von neuem an dem Patienten 7 zu befestigen sind bzw. einer Sterilisation bedürfen.

Wird die gesamte Vorrichtung ohne Patientenlagerungstisch 5 mobil ausgestaltet, so läßt sie sich auf einfachste Weise zur Patientenüberwachung einsetzen. Man fährt sie zu dem betreffenden Krankenbett und plaziert sie so, daß das gesamte Bett mit dem Patienten 7 im Einflußbereich der Spulen 2 ist. In einer Weiterentwicklung kann man einen rahmenförmigen Fuß- und Kopfteil des Bettes wählen und diesen als Spule 2 verwenden.

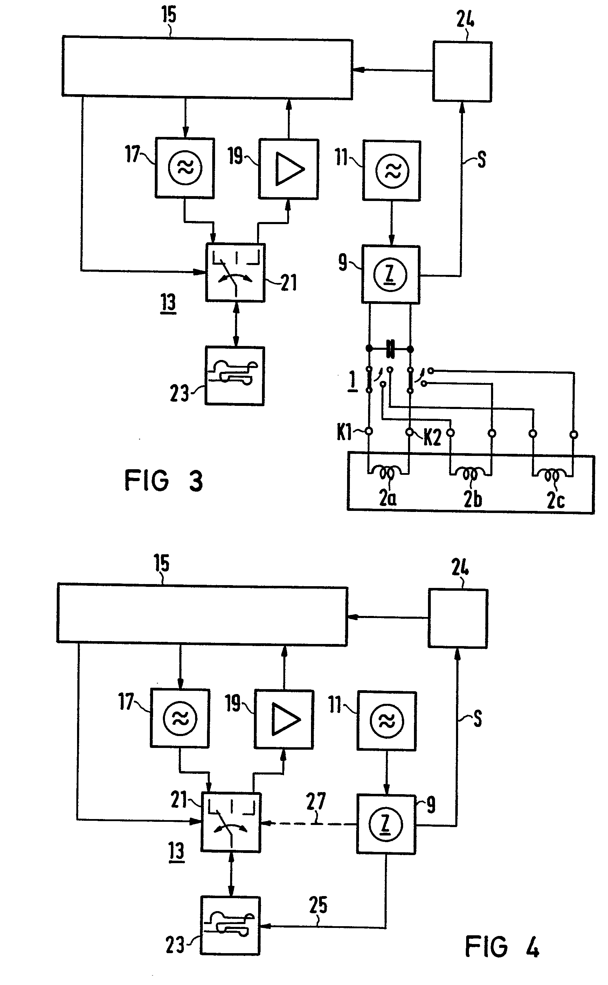

Figur 3 zeigt die Vorrichtung in Verbindung mit einem NMR-Gerät 13. Das NMR-Gerät 13 ist nur im Prinzip dargestellt durch eine Steuereinrichtung 15, einen Hochfrequenzsender 17, einen Hochfrequenzempfänger 19, einen Umschalter 21 und eine Hochfrequenzspule 23. Der Hochfrequenzsender 17 sendet über den Umschalter 21 Impulse auf die HF-Spule 23. Anschließend wird der Umschalter 21 auf den Hochfrequenzempfänger 19 geschaltet, um die von der Hochfrequenzspule 23 registrierten Spinlageänderungen im Patienten 7 zu empfangen und an die Steuereinrichtung 15 weiterzugeben. Funktion und Aufbau eines NMR-Gerätes 13 sind bekannt und sollen hier nicht näher erläutert werden. Wesentlich ist lediglich, daß das NMR-Gerät 13 eine Steuereinrichtung 15, einen HF-Sender 17, einen HF-Empfänger 19, und eine HF-Spule 23 beinhaltet.

Um die Bildaufnahmen bei Schnittbildern in der Herzgegend, in der Gegend des Zwerchfells oder anderen von Bewegungen beeinflußten Gebieten in ihrer Qualität zu verbessern, wird so vorgegangen, daß die Hochfrequenzimpulse immer dann auf die HF-Spule 23 gegegeben werden, wenn sich das betroffene Körperorgan in einer gleichen (äquivalenten) Bewegungsphase befindet.

Wie in Figur 3 dargestellt, geschieht dieses unter Zuhilfenahme der Vorrichtung 2, 9, 11 nach Figur 1. Das Meßsignal S wird der Steuereinrichtung 15 für die Bildaufnahme zugeführt. Über eine Triggerlogik oder Trigger-

0168691

schaltung 24 an der Steuereinrichtung 15 wird anhand des Meßsignales S festgelegt, wann die NMR-Meßsequenz für die Bildaufnahme gestartet werden kann.

Figur 3 zeigt noch einen weiteren Umschalter 24, mit dem wahlweise eine der Spulen 2a, 2b oder 2c, in den Schwingkreis 1 schaltbar ist. Die Spulenpaare sind entlang einer gemeinsamen Achse, die z.B. parallel zur Körperlängsachse des Patienten 7 verläuft, angeordnet. Auf diese Weise lassen sich interessierende Körperbereiche für die Messung der Bewegung herausgreifen.

Die HF-Meßspule kann auch durch eine separat vorhandene Oberflächenspule für den NMR-Signal-Empfang realisiert sein.

In der Figur 4 ist dargestellt, daß die HF-Spule 23 gleichzeitig die Funktion der Meßspule 2 übernehmen kann. Die Impedanz-Meßeinrichtung 9 ist nur über eine Leitung 25 mit der Hochfrequenzspule 23 verbunden. Es ist zweckmäßig, die Frequenz (oder aber die Amplitude) des hochfrequenten Dauersignals anders zu wählen, als die entsprechenden Werte für den NMR-Hochfrequenzimpuls. Alternativ hierzu ist es möglich, eine Leitung 27 von der Meßeinrichtung 9 direkt zum Umschalter 21 zu führen und dadurch zu bewirken, daß das Dauersignal zur Ermittlung der Organbewegungen ausgetastet wird, wenn die NMR-Hochfrequenzimpulse zur Ermittlung der Bilddaten ausgesendet werden.

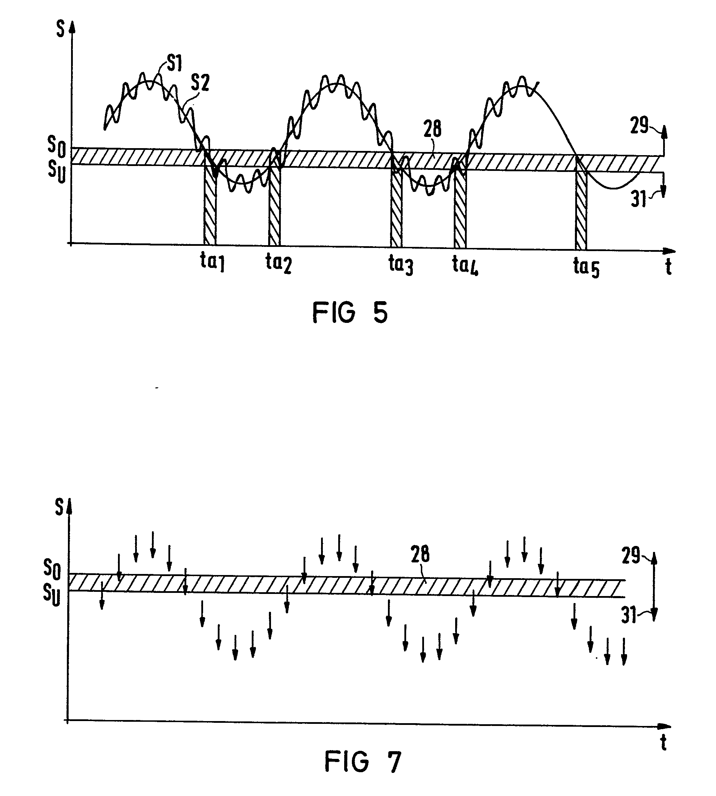

Die Figur 5 zeigt eine schematische Darstellung, wie die Triggerung des Bildauslösezeitpunktes in Abhängigkeit von der Organbewegung ablaufen kann. Das Meßsignal S setzt sich z.B. aus zwei Schwingungen S1 und S2 verschiedener Frequenz zusammen, die mit herkömmlichen Mitteln, wie mit einem Bandpaßfilter, selektiert werden können.
Das Meßsignal S2 repräsentiert z.B. die Bewegung der
Lunge und das Meßsignal S1 die Bewegung des Herzens. Für
das Meßsignal S2 entsprechend der Lungenbewegung werden
z.B. ein oberer Wert $S_o$ und ein unterer Wert $S_n$ festgelegt, die eine Bewegungsphase markieren, innerhalb welcher die Ausdehnungen der Lunge ähnlich sind. Die Werte
$S_o$ und $S_u$ schließen ein Bewegungsband 28 ein. Die Werte
$S_o$ und $S_u$ können nach Wahl in Richtung zweier Pfeile
29, 31 nach oben und/oder unten verschoben werden. Somit
kann der Bereich für den Auslösezeitpunkt festgelegt
werden. Das Band 28 läßt sich z.B. auf eine Phase in der
Gesamtbewegung einstellen, in welcher die Änderung der
Bewegung möglichst langsam erfolgt. Dem Bereich 28 zwischen den Meßsignalen $S_o$ und $S_u$ ist jeweils eine Auslösezeitspanne $ta_1$, $ta_2$, $ta_3$ ... usw. zugeordnet, innerhalb derer die Meßsequenz abläuft.

Analog kann für das Meßsignal S1, das die Herzmuskelbewegung darstellt, vorgegangen werden. Es bietet sich also ein weites Feld an Variationen, wie das Meßsignal S
zu nutzen ist. Außerdem gibt es einen weiten Anwendungsbereich, wie es zur Triggerung der Bildaufnahme im NMR-
Gerät 13 herangezogen werden kann.

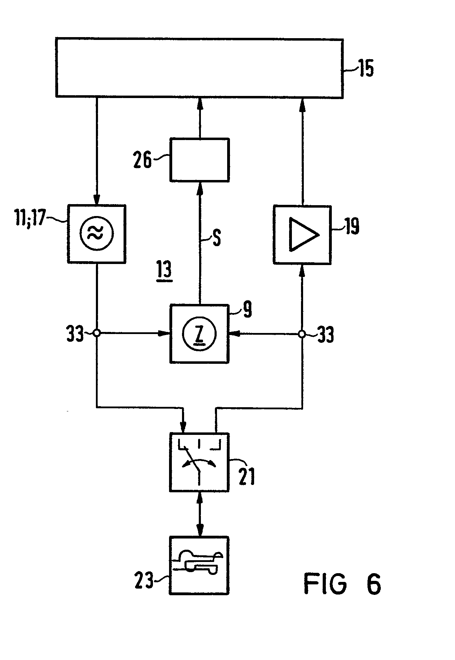

Die Figur 6 zeigt eine Variante, bei der weitgehend auf
die bestehenden Bauteile des NMR-Gerätes 13 zurückgegriffen wird. Es wird sowohl die HF-Spule 23 als Meßspule 2 als auch der HF-Sender 17 als Meßsender 11 verwendet. Einzige Modifizierung ist, daß zwischen dem HF-Sender 17 und dem Umschalter 21 sowie zwischen HF-Empfänger
19 und Umschalter 21 jeweils ein Knotenpunkt 33 gebildet
wird, an welchen die Meßeinrichtung 9 geschaltet ist.
Das Meßsignal S führt über eine Einrichtung 26 wiederum
in die Steuereinrichtung 15 für die Bildaufnahme. Bei dieser Vorrichtung wird entsprechend der Figur 7 die
Bewegungskurve des Organs nicht kontinuierlich über ein
Hochfrequenzdauersignal erfaßt, sondern diskret entsprechend der Repetitionszeit des NMR-Gerätes 17. Bei jedem
Senden und Empfangen des Hochfrequenzimpulses wird
gleichzeitig die Impedanz des Schwingkreises 1 inklusive
Patient 7 gemessen. Es entsteht so eine Vielzahl an Meßsignalen S, aus denen sich die Organbewegung zusammmensetzen läßt. Man kann sagen: Die Organbewegung wird mit
Hilfe der Meßeinrichtung 9 berührungslos abgetastet.

Es ist hierbei nicht möglich, die Bildaufnahme des NMR-
Gerätes 13 mit der Organbewegung zu steuern, da immer
erst hinterher, also nach bereits erfolgter Aufnahme
eines Bildpunktes, die Impedanz des Schwingkreises 1 bestimmt wird. Vielmehr wird im Nachhinein entschieden,
ob der aufgenommene Bildpunkt innerhalb des Bereiches 28
zwischen den Werten $S_o$ oder $S_u$ gelegen hat oder nicht.
Ist dieses der Fall, so wird er für die Zusammensetzung
des Gesamtschnittbildes verwendet; ist dieses nicht der
Fall, so wird er als Nutzsignal gesperrt.

Vorteil dieser Vorrichtung nach Figur 6 ist, daß wesentliche Elemente des NMR-Gerätes 13 gleichzeitig für die
Messung der Organbewegung mitverwendet werden können.
Zusätzlich sind nur noch die Bauelemente zum Aufbau des
Schwingkreises 1 und die Meßeinrichtung 9 nötig.

Die Signaländerung des HF-Schwingkreises kann, wie oben
dargestellt, zur Gewinnung einer Atemkurve herangezogen
werden, um die Bildaufnahme mit den Organbewegungen zu
synchronisieren.

Andererseits führen diese Impedanzänderungen durch die
Fehlanpassung des HF-Meßsenders an den HF-Schwingkreis

0168691

- 13 -      VPA 84 P 3247 E

zu einer Verschlechterung des Signal/Rausch-Verhältnisses bei der NMR-Messung. Diese Verschlechterung kann durch entsprechendes Nachregeln der Impedanz des HF-Schwingkreises vermieden werden.

Dieses Nachregeln führt einerseits zu einer besseren Qualität der NMR-Bilder. Andererseits ist die Regelspannung ein Maß für Organbewegungen im Meßfeld und kann, wie oben beschrieben, wieder benutzt werden, um die Bilddatenerfassung zu steuern.

Zum Begriff der Hochfrequenz ist folgendes zu sagen. Bei NMR-Geräten hängt die Sendefrequenz von der Feldstärke des Magnetfeldes ab. Heute gebräuchliche Magnetfeldstärken liegen im Bereich von 0,1 - 2,5 Tesla. Das würde eine Hochfrequenz im Bereich von 3 bis 106 MHz (pro Tesla ca. 42,6 MHz) für den Nachweis von Wasserstoffprotonen ergeben. Auf dem Markt erhältliche NMR-Geräte arbeiten mit einer Magnetfeldstärke von 0,3 - 1,5 Tesla. Das ergibt eine Hochfrequenz im Bereich von 12 bis 64 MHz für den Nachweis der Wasserstoffprotonen. Für den Nachweis anderer Elemente sind entsprechend niedrigere oder höhere Hochfrequenzen einzusetzen; z. B. bei Phosphor niedrigere.

19 Patentansprüche
7 Figuren

Patentansprüche

1. Vorrichtung zur berührungslosen Messung der Bewegungen an einem ruhenden Patienten, insbesondere zur Messung von Organbewegungen, die durch Atmung oder Herzschlag verursacht sind, d a d u r c h   g e k e n n - z e i c h n e t ,   daß eine mit einem HF-Meßsender (11; 17) verbundene HF-Meßspule (2) Bestandteil eines HF-Schwingkreises (1) ist, daß an der HF-Meßspule (2) ein zur Aufnahme des Patienten (7) bestimmter Raum (3) vorgesehen ist, und daß eine Meßeinrichtung (9) an dem Schwingkreis (1) angeschlossen ist, an deren Signalausgang ein Meßsignal (S) abgreifbar ist, welches dann, wenn der Patient (7) in den besagten Raum (3) eingebracht ist, eine Änderung der Dämpfung des Schwingkreises (1) aufgrund einer Masseverschiebung des Patienten (7) anzeigt.

2. Vorrichtung nach Anspruch 1, d a d u r c h   g e - k e n n z e i c h n e t ,   daß sie einem NMR-Gerät (13) mit einer HF-Sende- und -Empfangsspule (23) und mit einem HF-Sender (17) zugeordnet ist (Fig. 3, 4 und 6).

3. Vorrichtung nach Anspruch 2, d a d u r c h   g e - k e n n z e i c h n e t ,   daß die HF-Sende- und -Empfangsspule (23) zugleich die HF-Meßspule (2) ist (Fig. 4 und 6).

4. Vorrichtung nach Anspruch 1, d a d u r c h   g e - k e n n z e i c h n e t ,   daß sie einem NMR-Gerät mit getrennter HF-Sendespule und HF-Empfangsspule zugeordnet ist.

5. Vorrichtung nach Anspruch 4, d a d u r c h   g e - k e n n z e i c h n e t ,   daß die HF-Empfangsspule zugleich die HF-Meßspule (2) ist.

6. Vorrichtung nach Anspruch 2 oder 3, d a d u r c h g e k e n n z e i c h n e t , daß der HF-Meßsender (11) zugleich der HF-Sender (17) des NMR-Gerätes (13) ist (Fig. 6).

7. Vorrichtung nach Anspruch 2, d a d u r c h g e - k e n n z e i c h n e t , daß mehrere HF-Meßspulen (2a, 2b, 2c), die wahlweise in den HF-Schwingkreis (1) schaltbar sind, räumlich nebeneinander entlang einer gemeinsamen Achse angeordnet sind (Fig. 3).

8. Vorrichtung nach Anspruch 3 und 6, d a d u r c h g e k e n n z e i c h n e t , daß die Repititionszeit für Senden und Empfangen der HF-Sende- und -Empfangsspule (23) klein gewählt ist im Vergleich zur Wiederholzeit der zu untersuchenden Bewegung (Fig. 6).

9. Vorrichtung nach einem der Ansprüche 1 bis 7, d a - d u r c h g e k e n n z e i c h n e t , daß vom HF-Meßsender (11) auf die HF-Meßspule (2) ein HF-Dauersignal gegeben ist (Fig. 3 und 4).

10. Vorrichtung nach Anspruch 9, d a d u r c h g e - k e n n z e i c h n e t , daß bei Anwendung im NMR-Gerät (13) das HF-Dauersignal während der Sende- und Empfangszeiten der HF-Sende- und -Empfangsspule (23) ausgetastet ist.

11. Vorrichtung nach einem der Ansprüche 2 bis 10, ausgenommen Anspruch 6, d a d u r c h g e k e n n - z e i c h n e t , daß die HF-Meßspule (2) vom HF-Meßsender (11) mit einem Sendesignal beaufschlagt ist, dessen Frequenz von der des HF-Senders (17) des NMR-Gerätes (13) unterschiedlich ist (Fig. 3 und 4).

12. Vorrichtung nach einem der Ansprüche 2 bis 11, wobei das NMR-Gerät eine Steuereinrichtung für die Bildaufnahme aufweist, d a d u r c h   g e k e n n z e i c h - n e t , daß das von der Meßeinrichtung (9) abgegebene Meßsignal (S) über eine Triggerschaltung (24) zur Steuereinrichtung (15) für die Bildaufnahme geführt und dort zur Triggerung der Bildaufnahme vorgesehen ist (Fig. 3 und 4; Fig. 5).

13. Vorrichtung nach einem der Ansprüche 2 bis 11, d a d u r c h   g e k e n n z e i c h n e t , daß das von der Meßeinrichtung (9) abgegebene Meßsignal (S) zu einer Logikschaltung (26) geführt ist, die zur Freigabe oder Sperrung bereits gewonnener Bilddaten vorgesehen ist (Fig. 6).

14. Vorrichtung nach einem der Ansprüche 2 bis 13, d a d u r c h   g e k e n n z e i c h n e t , daß das Meßsignal (S) einer Einrichtung zur Feststellung von unrhythmischen Bewegungsartefakten zugeführt ist, und daß das Ausgangssignal dieser Einrichtung zur Sperrung der Bilddaten bei Erkennung eines Artefaktes vorgesehen ist (Fig. 3 und 4).

15. Vorrichtung nach Anspruch 13, d a d u r c h   g e k e n n z e i c h n e t , daß die Logikschaltung (26) eine Einrichtung zur Feststellung gleicher Bewegungsphasen ist, an der ein vorgegebenes Bewegungsband (28) eingestellt ist, und daß das Ausgangssignal dieser Einrichtung zum Freigeben der Bilddaten gleicher Bewegungsphasen vorgesehen ist (Fig. 7).

16. Vorrichtung nach einem der Ansprüche 1 bis 15, d a d u r c h   g e k e n n z e i c h n e t , daß der HF-Meßsender (11) eine Sendefrequenz von ca. 20 MHz besitzt.

17. Vorrichtung nach einem der Ansprüche 1 bis 15, d a d u r c h   g e k e n n z e i c h n e t ,  daß der Schwingkreis (1) auf eine Impedanz von etwa 50 Ohm abgeglichen ist, so daß er an den HF-Meßsender (11) leistungsmäßig abgepaßt ist.

18. Vorrichtung nach einem der Ansprüche 1 bis 17, d a d u r c h   g e k e n n z e i c h n e t ,  daß die Impedanz des HF-Schwingkreises (1) mit Hilfe eines Regelkreises konstant gehalten ist.

19. Vorrichtung nach Anspruch 18,   d a d u r c h   g e k e n n z e i c h n e t ,   daß das Ausgangssignal des Regelkreises, das ein Maß für die Bewegungen des Patienten ist, zur Triggerung des NMR-Gerätes vorgesehen ist.

FIG 1

FIG 2

FIG 3

FIG 4

FIG 5

FIG 7

FIG 6

Europäisches
Patentamt

EUROPÄISCHER RECHERCHENBERICHT

0168691
Nummer der Anmeldung

EP 85 10 7986

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | IEEE TRANSACTIONS ON BIO-MEDICAL ENGINEERING, Band BME-15, Nr. 4, Oktober 1968, Seiten 266-278, IEEE, New York, US; P.P. TARJAN u.a.: "Electrodeless measurements of the effective resistivity of the human torso and head by magnetic induction" * Seiten 266-273, die Abschnitte "Abstract", "Equipment and measurements procedures" und "Results: Human torso" * | 1 | A 61 B 5/10<br>A 61 B 5/05<br>G 01 N 24/04 |
| X | IEEE TRANSACTIONS ON BIO-MEDICAL ENGINEERING, Band BME-28, Nr. 10, Oktober 1981, Seiten 702-710, IEEE, New York, US; D.L. WILSON u.a.: "Physical principles of the displacement cardiograph including a new device sensitive to variations in torso resistivity" * Seite 702, "Abstract"; Seiten 704-710, die Abschnitte "Methods and Procedures", "Results" und "Discussion". * | 1 | |
| A | EP-A-0 096 487 (PICKER INTERNATIONAL INC.) * Zusammenfassung; Seite 3, Zeilen 4-15; Seite 4, Zeile 26 - Seite 5, Zeile 8; Seite 7, Zeilen 13-27; Seite 8, Zeilen 14-16; Seite 9, Zeile 13 - Seite 10, Zeile 11; Abbildungen 1-5 * | 1,2,13,19 | |

RECHERCHIERTE SACHGEBIETE (Int. Cl.4)

A 61 B
G 01 N

--- -/-

Der vorliegende Recherchenbericht wurde für alle Patentansprüche erstellt.

| Recherchenort | Abschlußdatum der Recherche | Prüfer |
|---|---|---|
| DEN HAAG | 14-10-1985 | RIEB K.D. |

Europäisches
Patentamt

**EUROPÄISCHER RECHERCHENBERICHT**

0168691

Nummer der Anmeldung

EP 85 10 7986

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | RÖNTGENSTRAHLEN, Nr. 49, 1983, Seiten 34-41, DE; J. HEINZERING u.a.; "Technische Fortschritte in der NMR-Tomographie" * Seite 40, mittlere Spalte, Zeilen 5-22.* ----- | 15,19 | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| DEN HAAG | 14-10-1985 | RIEB K.D. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82